# EUROPEAN PATENT SPECIFICATION

(11) **EP 1 882 487 B1**
(45) Date of publication and mention of the grant of the patent: **23.03.2011**
(21) Application number: 07111154.6
(22) Date of filing: 27.06.2007
(51) Int. Cl.: A61M 15/00, A61M 11/00

(54) **Inhaler**
Inhalator
Inhalateur

(30) Priority: 24.07.2006 JP 2006200189; 08.06.2007 JP 2007152215
(43) Date of publication of application: 30.01.2008
(73) Proprietor: CANON KABUSHIKI KAISHA, Ohta-ku Tokyo 146-8501 (JP)
(72) Inventor: Kaneko, Hideki, Tokyo Tokyo 146-8501 (JP)
(74) Representative: TBK-Patent

(56) References cited:
- EP-A- 1 201 258
- WO-A-01/37910
- JP-A- 2004 350 985
- US-A- 5 036 840
- US-A- 5 613 489

## Description

### BACKGROUND OF THE INVENTION

### Field of the Invention

This invention relates to an inhaler having the features of the preamble of claim for inhaling aerosol medicines or the like.

### Description of the Related Art

In virtue of inhalers with which users inhale to take medicines, treatment for users is being materialized which enables utilization of information data bases such as electronic medical records. Such inhalers are also portable terminals having in combination i) a memory means which stores information concerned with each individual user, inclusive of information on user's medical records and prescription and ii) an atomizer which sprays a medicine made into an aerosol to make the user inhale it. It also has an atomization control means which controls the inhaler in accordance with inspiration or inhalation profiles of the user to atomize the medicine so that the user can inhale the medicine in conformity with the information on prescription (see WO95/01137 and WO02/04043).

Now, in medical treatment works, the reproducibility of the efficacy of medicine is especially important. Where a medicine is taken by using the above inhaler (i.e., in the case of administration through lungs), it is important to secure the reproducibility of the efficacy of medicine every time a patient inhales the medicine, that is, when inhaling the medicine in the same quantity, to take up the medicine always in the same quantity. This is because the same efficacy of medicine can always be achieved as long as the medicine having been inhaled is taken up in a constant quantity. If the medicine has a different efficacy every time the patient inhales it, there is a possibility that, in the case of, e.g., insulins, such a difference leads to a serious problem such as hypoglycemia.

In the efficacy of medicine in the administration through lungs, it is known that the particle diameter of the medicine is important. This is because deposition-to-lung rate of the medicine is deeply concerned with medicine particle diameter. Where medicine particles inhaled are large, such medicine particles can not turn at body portions turning in inhalation passages (e.g., the throat and the bronchial tubes), to come to collide against and adhere to their walls, so that they can not reach the lungs. The medicine having adhered to the bronchial tubes and so forth is taken up at a lower speed than a case in which it is taken up at lung alveoli, and also some of such medicine may be discharged when, e.g., you cough out phlegm. Hence, there is a possibility that the medicine is taken up in a small quantity. In addition, if the medicine having been inhaled has too small particle diameter, the medicine is extracoporeally discharged by exhalation without being deposited in lungs. It is generally said that the medicine may preferably have a particle size of about 3 µm in order to reach lungs and come deposited there. More specifically, the higher proportion the medicine particles of about 3 µm in diameter have among medicine particles inhaled, the higher deposition-to-lung rate, i.e., the higher efficacy of medicine can be achieved. Accordingly, where a user inhales a medicine by using the inhaler, it is important for the user to inhale a medicine having a constant particle diameter (about 3 µm) in whatever environment the inhaler is used.

Where the medicine having a constant particle diameter is ejected in a constant quantity from a medicine ejection part and intracoporeally led to the user in an environment of constant open-air temperature and open-air humidity, the particle diameter may change always at a constant level, and hence the user, a patient, can always inhale the medicine having a constant particle diameter. However, if the open-air temperature and open-air humidity change every time the patient uses the inhaler, the level of changes in particle diameter of the medicine the patient inhales becomes larger or smaller.

Where the medicine is liquid, its evaporation may greatly be accelerated when the temperature is extremely high or when the humidity is extremely low, as compared with common environments. As the result, there is a possibility that, before the medicine having been atomized and having a constant particle diameter reaches the patient's mouth, it comes to have a small particle diameter which is undesirable in view of the rate of deposition of medicine to lungs (deposition-to-lung rate). Where on the other hand the medicine is solid, mutual agglomeration of medicine particles may greatly be accelerated when the humidity is extremely high. That is, there is a possibility that, before the medicine having been atomized and having a constant particle diameter reaches the patient's mouth, it comes to have a large particle diameter which is undesirable in view of the rate of deposition of medicine to lungs (deposition-to-lung rate).

As a method by which the particle diameter of a medicine the patient inhales is kept constant, a technique is available in which an air flow path through which the medicine is led to the user is provided therein with a heater to control the particle diameter of the medicine (see W02000/00244). However, the heater consumes electricity and hence this leads the device to have a large size. Such an inhaler is expected to be made compact so as to be usable without care of place, and hence is not preferable as a compact device the patient can carry.

The inhaler may otherwise be provided therein with a portion where exhaled air of a patient is stored (an exhaled-air storing portion), and the medicine is atomized into that portion to make the patient inhale the air in that portion so that the particle diameter of the medicine the patient inhales can be kept always constant (see JP No. 2004-350985). In this case, however, there is much concern that the medicine adheres to the walls of the exhaled-air storing portion. A measure to prevent the medicine from adhering to the walls is taken in this JP No. 2004-350985, but has not been satisfactory. Once the medicine has adhered to the walls of the exhaled-air storage portion, the medicine to be inhaled may decrease. This not only results in a poor efficiency of treatment, but also is undesirable in view of sanitation.

EP 1 201 258 A2 shows a device for optimizing a dose precipitation in an inhalation unit for the administration of medicine, which comprises a container made of two stiff members for storing exhaust air of a user, a flow path member for guiding air, an ejection part for ejecting medicine into the air and a suction part for enabling a user to inhale the thus created air-medicine-mixture. Furthermore, a control unit is connected to the container in order to detect the amount of exhaust air and to match the medicine amount to be ejected to this exhaust air amount.

WO 01/37910 A1 describes a device for assisted inhalation, wherein an air-medicine-mixture is supplied to a patient in need of medication, when the patient is not capable of inhaling by his own strength.

### SUMMARY OF THE INVENTION

The present invention has been made taking account of the above unsettled problem the background art has had. Accordingly, an object of the present invention is to provide an inhaler with which the medicine having a constant particle diameter can always be inhaled and the same efficacy of medicine can always be achieved.

This object is achieved by an inhaler comprising the features of claim 1 of the invention.

The inhaler of the present invention is an inhaler with which a user inhales a medicine through a suction port and which has the following: a gas holding part in which exhaled air of the user or other air is to be stored; an air flow path which is connected with the gas holding part and guides the medicine to the suction port by inhalation of the user; and a medicine ejection part from which the medicine is to be ejected to the interior of the air flow path when the user inhales through the air flow path the exhaled air or other air stored in the gas holding part.

The inhaler of the present invention does not make it come about that the medicine inhaled adheres to inner walls or the like of the air flow path to decrease, and also enables the user to always inhale the medicine having a constant particle diameter. As the result, the reproducibility of the efficacy of medicine can be ensured every time the patient inhales the medicine.

Other features and advantages of the present invention will be apparent from the following description taken in conjunction with the accompanying drawings, in which like reference characters designate the same or similar parts throughout the figures thereof.

Further features of the present invention will become apparent from the following description of exemplary embodiments with reference to the attached drawings.

### BRIEF DESCRIPTION OF THE DRAWINGS

Fig. 1 is a sectional view showing an inhaler according to a first embodiment of the present invention.

Fig. 2 is a view showing a state where exhaled air of a patient is stored in a container 2 in what is shown in Fig. 1.

Fig. 3 is a sectional view showing an inhaler according to a second embodiment of the present invention.

Fig. 4 is a view showing a state where exhaled air of a patient is stored in a cylinder 7 in what is shown in Fig. 3.

Fig. 5 is a sectional view showing an inhaler according to a third embodiment of the present invention.

Fig. 6 is a view showing a state where a container 2 in which air having constant temperature and humidity is stored is set attached to a flow path member 3 in what is shown in Fig. 5.

### DESCRIPTION OF THE EMBODIMENTS

Preferred embodiments of the present invention will now be described in detail with reference to the accompanying drawings.

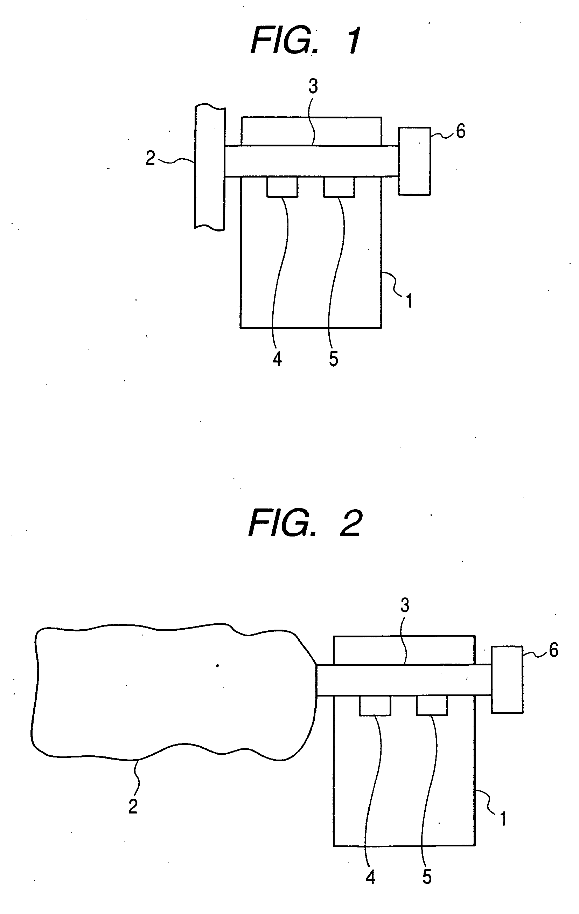

Fig. 1 sectionally illustrates an inhaler which is a first embodiment of the present invention. The inhaler according to the first embodiment of the present invention has a device main body 1, a container 2 having flexibility which is a gas holding part in which exhaled air of a user (patient) or other air conditioned to have constant temperature and humidity is stored, a flow path member 3 one end of which is connected with the container 2 and which forms an air flow path through which a medicine is guided to a suction port 6 by inhalation of the user, a medicine ejection part 4 for ejecting (atomizing or spraying) the medicine therefrom, and a pressure sensor 5 for sensing negative pressure produced in the air flow path by the inhalation of the patient.

Medicine

In the present specification, the "medicine" includes any physiological or pharmacological active substances having local or systemic effects on patients. The active substances to be administered include antibiotics, antiviral substances, antiepileptics, analgesics, antiphlogistics and bronchodilators, and also include viruses, any of which may be inorganic or organic compounds. The medicine referred to in the present specification also include medicines acting on peripheral nerves, adrenergic receptors, cholinergic receptors, skeletal muscles, the circulatory system, smooth muscles, the vascular system, synapse sites, nerve effector synapses, the endocrine system and hormone system, the immunological system, the genital system, the skeletal system, the autacoid system, the digestive system and excretory system, the histamine system and the central nervous system. For example, it may be selected from polysacchalides, steroids, hypnotics and sedatives, psychoactivating agents, tranquilizers, antispasmodics, muscle reluxants, antiperkinsonics, analgesics, antiphlogistics, muscle contractors, anti-infectious agents, antibiotics, antimicrobial agents, antimalarial agents, hormonal reactants inclusive of contraceptives, sympathetic- nerve stimulants, polypeptides, proteins capable of inducing physiological action, diuretics, lipid regulators, anti-androgenic agents, vermicides, neoplasmic agents, antineoplasmic agents, blood sugar reducers, nutrients and supplements, growth supplements, fats, anti-enteritis agents, electrolytes, vaccines, and diagnostic agents.

Examples of active substances useful in the present invention include, but are not limited to, insulins, calcitonins, erythropoietins (EPO), factor VIII, factor IX, Ceredase, Cerezyme, cyclosporins, granular colony stimulation factors (GCSF), α-1-proteinase inhibitors, elcatonins, GMCSF (granulocyte-macrophage colony-stimulating factors, growth hormones, HGH (human growth hormones), GHRH (growth hormone- releasing hormone), heparins, LMWH (low-molecular weight heparins), interferon α, interferon β, interferon y, interleukin 2, LHRH (luteinizing hormone- releasing hormone), somatostatin, somatostatin analogues containing octreotide, vasopressin analogues, FSH (follicle-stimulating hormones), insulin-like growth factors, insulintropin, interleukin 1 receptor antagonists, interleukin 3, interleukin 4, interleukin 6, M-CSF (macrophase colony-stimulating factors), nerve growth factors, PTH (parathyroid hormones), thymosin α1, IIb/IIIa inhibitors, α1 antitrypsins, respiratory system symplast virus antibodies, CFTR (cystic fibrous transmembrane conductance regulator) genes, deoxyribonucleases, BPI (bacterial/permeability- increasing protein), anti-CMV(cytomegalovirus) antibodies, interleukin 1 receptors, 13 cis-retinoic acid, pentamidine isothionate, albuterol sulfate, metaproterenol sulfate, beclometasone dipropionate, triamcinolone acetamide, budesonide acetonide, ipratrobium bromide, flunisoride, fluticasone, cromolyn sodium, nicotine, lung surface-active agents, amphotericin B, ciprofloxasin, gentamycins, tobramycins, ergotamine tartrate, and analogues, agonists and antagonists of the foregoing. It may further have structure of a nucleic acid appearing as Beer's nucleic acid molecules; a nucleic acid relating to, or incorporated in, viral vectors, related viral particles, lipids or lipid-containing materials; and other nucleic acid of a type suited for transfection or transformation of plasmid DNA or RNA or cells, in particular, cells of lung alveoli regions. The above substances may have various forms of, e.g., soluble or insoluble charged or non-charged molecules, and components of molecular synthetic products or pharmacyologically acceptable bases. The above active substances (reactants) may be naturally occurring molecules, may be produced by recombination, or may be analogues of active substances (reactants) having naturally occurred or having been produced by recombination and at least one amino acid of which has been added or deleted. The above substances may further include attenuated viruses or inactivated viruses suited for use as vaccines.

A liquid medicine used in the present invention refers to a medicine in the form of a liquid or a liquid medium containing a medicine. The liquid medicine may contain any desired additive(s). The medicine in a liquid may be in the state of any of dissolution, dispersion, emulsification, suspension and slurry, and may more preferably stand homogeneous in the liquid.

In the case when the liquid medicine is used as the medicine, the chief medium of the liquid may preferably be water or organic matter. Taking account of the fact that the medicine is administered to living bodies, it is preferable that water is the chief medium.

### Medicine Atomizing Means

In the present invention, the medicine ejection part (ejection head) uses the principle of an inkjet system.

In the case when the thermal jet system is used, size precision and reproducibility of: nozzle diameter of the ejection nozzle, calories of heat pulses utilized for ejection, a micro-heater as the electrothermal transducer can be enhanced in respect of individual liquid ejection units. Hence, a narrow droplet diameter distribution can be achieved. In addition, production cost for the head can be so low that this system is highly adaptable to compact devices which require frequent replacement of heads. Accordingly, the medicine ejection device of such a thermal jet system is particularly preferred when the medicine ejection device is required to have portability and convenience.

The ejection head may be provided integrally with a medicine reservoir to set up a medicine ejection cartridge, or may be set up as a member separate from the medicine reservoir.

According to the inhaler of the present invention, the patient can always inhale the medicine having a constant particle diameter. Hence, the reproducibility of the efficacy of medicine can be achieved without decreasing the medicine to be inhaled, without care of any contamination due to adhesion of the medicine and without regard to use environment at the time of inhalation.

The inhaler of the present invention is so set up that the user can carry it, and has a memory means (a memory) which stores information concerned with each individual user, inclusive of information on user's medical records and prescription. Then, it is an inhaler which makes the user inhale the medicine and is provided with a means for atomizing or nebulizing a medicine having a high uniformity in particle size. It is so designed that the user can efficiently and sanitarily inhale the medicine through the suction port (a mouthpiece) in accordance with the information on prescription. Herein, in the present specification, the "flow path member" means a member that forms an air flow path which is a medicine passage extending in the inhaler from a medicine-ejected part to the suction port. That is, the space formed inside the flow path member corresponds to the "air flow path".

In conventional inhalers, it has commonly be so set up that one end of the flow path member 3 is connected with the suction port 6 and, on the other end thereof, an opening (open-air intake) is provided so that air streams can be formed in the flow path member 3 when the user inhales the medicine through the suction port 6. In such an inhaler, the air streams on which the medicine is transported is the open air, and hence there has been a possibility of changes in particle diameter of the medicine depending on open-air environmental conditions (such as temperature and humidity).

In the present invention, the flow path member 3 and the suction port 6 may connectably be set up as separate members, or may integrally be set up.

Embodiment 1

Fig. 1 sectionally illustrates the inhaler which is a first embodiment of the present invention. The inhaler according to the first embodiment of the present invention is characterized in that a container 2 serving as a gas holding part in which the exhaled air of a user or other air is stored is provided at the part that has conventionally been an opening. The container 2 is connected with the flow path member 3 in the state of being folded up and is so designed that a patient can send his or her exhaled air to the container 2 from a suction port 6 through the air flow path. Further, the inhaler is so set up that a pressure sensor 5 is provided in the air flow path to detect negative pressure produced in the air flow path by the inhalation of the patient and that the medicine can be atomized from an ejection head 4 to the interior of the mouthpiece in synchronization with such detection. It is preferable for the flow path member 3 to be detachably attached to the device main body 1.

The container 2 may have such a flexibility so that it may inflate when the user blows and it may crush when the user inhales. Materials for the container 2 may include paper and vinyl. Aluminum foil, ethylene-vinyl alcohol copolymer resin (EVOH), polyvinylidene chloride (PVDC) and butyl rubber, which have a low gas permeability, are preferable in order to make it easy to keep temperature and humidity constant in the container. There are no particular limitations on how the container be folded. It may be folded irregularly, in bellows structure, or spirally. Such a foldable container 2 may preferably be in a size set appropriately depending on age, build or figure, lung capacity, and so forth.

Once the patient blows through the suction port, the container 2 inflates, thus the exhaled air can be stored therein (Fig. 2). The exhaled air of the patient has a temperature of about 37°C and a humidity of about 95% without regard to the outside temperature and humidity, thus air having constant temperature and humidity can be stored in the container 2. Once the patient begins to inhale, the pressure sensor 5 provided in the air flow path detects the inhalation, and, in synchronization therewith, the medicine is atomized into the air flow path from the ejection head 4 as a medicine atomizing means. Device control including the driving of the ejection head is performed by a controller (CPU) (not shown) provided in the main body 1. The controller sends drive signals to the ejection head upon detection of the negative pressure by the pressure sensor 5. The medicine is atomized into air streams of the air having constant temperature and humidity which has been stored in the container 2. That is, a liquid-droplet medicine having a constant particle diameter and having been atomized in the air flow path is always atomized into the air having constant temperature and humidity without regard to the temperature and humidity in surroundings of the device. Hence, the amount of evaporation of the medicine is also always constant while it is transported on the air streams to come to reach the suction port 6. Thus, the patient can inhale the medicine having a constant particle diameter without regard to the temperature and humidity in surroundings of the device.

In what is shown in Figs. 1 and 2, the pressure sensor 5 is disposed on the side nearer to the suction port 6 than the ejection head 4. There, however, are no particular limitations on the position of the pressure sensor 5 in the air flow path. Also, it is a preferable embodiment that the pressure sensor 5 is disposed on the side upstream of the air streams more than the ejection head 4 so as not to cause adhesion of the medicine in the air flow path.

Embodiment 2

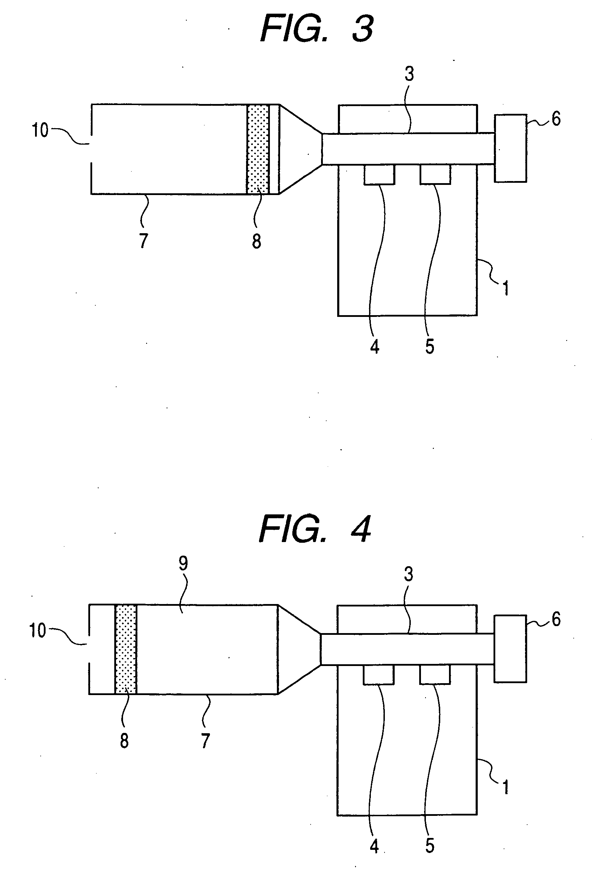

Figs. 3 and 4 illustrate a second embodiment in which a cylinder 7 is used as the gas holding part in place of the container 2 that is crushable upon inhalation of the user in Embodiment 1. In the state a movable part 8 of the cylinder 7 is on one side end of a flow path member 3 as shown in Fig. 3, a patient blows with a suction port 6 (mouthpiece) in his or her mouth, whereupon the air in the cylinder 7 escapes through its opening (air hole), so that the movable part 8 moves through the interior of the cylinder 7 and the exhaled air is stored in an exhaled-air storing portion 9 to come into the state shown in Fig. 4. The exhaled air stored in the exhaled-air storing portion 9 has substantially constant temperature and humidity of about 37°C and about 95%, respectively, without regard to the surrounding temperature and humidity. Thereafter, the patient begins to inhale, whereupon the air kept at constant temperature and humidity which has been stored in the exhaled-air storing portion 9 flows into the mouth of the patient, where a pressure sensor 5 detects inhalation. On the basis of signals having been sent from the negative pressure sensor 5, an ejection head 4 of a thermal jet system starts to atomize the medicine in the air flow path.

The medicine having a constant particle diameter which has always been atomized in the air flow path stands atomized into the air having constant temperature and humidity without regard to the temperature and humidity in surroundings of the device, and hence the amount of evaporation of the medicine is also always constant. Thus, the patient can always inhale a liquid droplet medicine having a constant particle diameter without regard to the temperature and humidity in surroundings of the device.

Embodiment 3

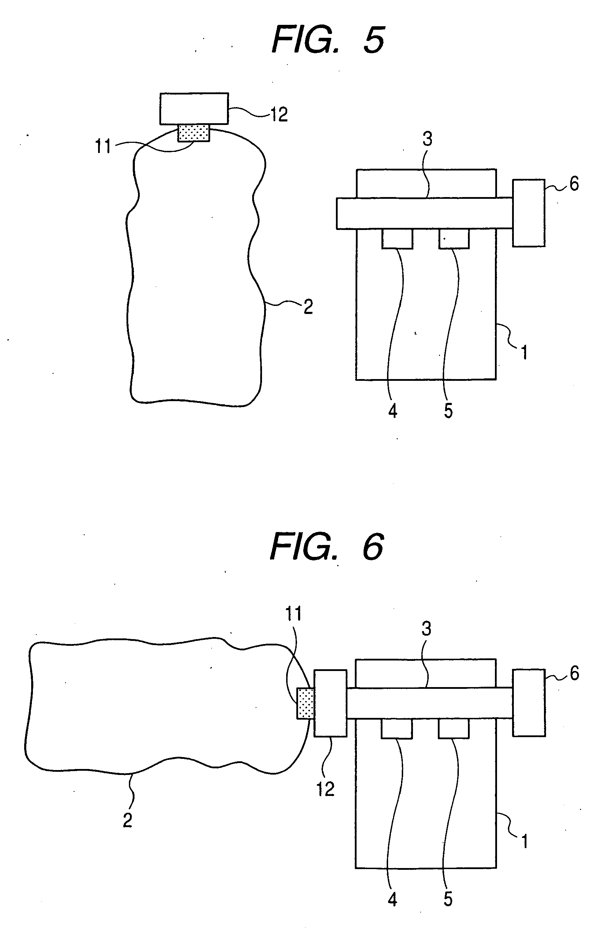

Figs. 5 and 6 are sectional views of an inhaler which illustrate a third embodiment of the present invention. A container 2 in this embodiment is a container made of butyl rubber and having a volume of 500 ml. It has an enclosure mechanism 11 and an adapter 12. The container 2 is a paper bag which is crushable upon inhalation of a user. The container 2 can be attached to a flow path member 3 and detached from the flow path member 3, by means of the adapter 12. That is, the container 2 is detachably attached to the flow path member 3. In the state the container 2 is detached from the flow path member 3 as shown in Fig. 5, air is kept enclosed in the air bag 2 in a constant-temperature or constant humidity environment. The air may be enclosed by, e.g., using a gas injector from a gas cylinder holding therein air having constant temperature or constant humidity, or may be enclosed by sending user's exhaled air therein. Environmental conditions of the air kept enclosed previously in the container 2 may preferably be set at average values of the temperature and humidity of an environment where the inhaler is supposed to be used, in order to make the conditions less differ on the average from the surrounding temperature and humidity considered in the environment where the inhaler is supposed to be used.

When the air is enclosed, it is necessary to enclose air at least the temperature or humidity of which is constant, and it is preferable to enclose air both the temperature and humidity of which are constant.

Immediately before inhalation by the patient, the container 2 in which the air is previously stored in a constant-temperature or constant-humidity environment is attached to the flow path member 3 by means of the adapter 12, thus the inhaler comes into the state shown in Fig. 6. Thereafter, the enclosure mechanism 11 is unlocked and the patient begins to inhale, whereupon the air kept at constant temperature and humidity which has been stored in the container 2 flows into the mouth of the patient, where a pressure sensor 5 detects inhalation. On the basis of signals having been sent from the pressure sensor 5, an ejection head 4 of a thermal jet system starts to atomize the medicine in the air flow path.

The medicine having a constant particle diameter which has always been atomized in the air flow path stands atomized into the air having constant temperature and humidity without regard to the temperature and humidity in surroundings of the device, and hence the amount of evaporation of the medicine is also always constant. Thus, the patient can always inhale a liquid droplet medicine having a constant particle diameter without regard to the temperature and humidity in surroundings of the device.

Table 1 given at the end shows results obtained by examining particle diameters measured when water droplets of 3.0 µm in diameter which have been formed by atomization in respective environments come out of a mouthpiece. Sectional area of the mouthpiece was set to be 100 mm²; distance from the atomization part to the mouthpiece outlet, 0.04 m; and flow rate of air streams in the air flow path, 20 m/s. The atomization was so carried out that the liquid droplets were formed at a frequency of 1,000,000 droplets/s.

As shown in Table 1, the particle diameter of liquid droplets when coming out of the mouthpiece differs greatly depending on environment. If the particle diameter of liquid droplets inhaled differs, the rate of deposition of medicine to lungs differs every time the medicine is inhaled, to make the reproducibility of the efficacy of medicine not achievable. Where the atomization of liquid droplets is carried out into the air having always constant temperature and humidity without regard to the temperature and humidity in surroundings of the device, the liquid droplets can always have a constant particle diameter, and hence the reproducibility of the efficacy of medicine can be achieved.

**Table 1**

| | | | |
|---|---|---|---|
| Temperature | Humidity | Particle diameter of medicine when atomized | Particle diameter of droplets when coming out of mouthpiece |
| 10°C | 90% | 3.00 µm | 2.98 µm |
| 25°C | 50% | 3.00 µm | 2.80 µm |
| 40°C | 10% | 3.00 µm | 2.37 µm |

The present invention is not limited to the above embodiments and various changes and modifications can be made within the scope of the present invention. Therefore, to apprise the public of the scope of the present invention, the following claims are made.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
To make a user inhale a medicine having a constant particle diameter, an inhaler with which the user inhales the medicine through a suction port has a gas holding part in which exhaled air of the user or other air is to be stored an air flow path which is connected with the gas holding part and guides the medicine to the suction port by inhalation of the user and a medicine ejection part from which the medicine is to be ejected to the interior of the air flow path when the user inhales through the air flow path the exhaled air or other air stored in the gas holding part.

## Claims

1. An inhaler for inhaling medicine through a suction port, the inhaler comprising:
the suction port (6);
a container (2) for storing therein exhaled air of a user or other air;
a flow path member (3) which is connected to the container (2) and forms an air flow path for guiding the medicine to the suction port (6) by inhalation of the user; and
a medicine ejection part (4) for ejecting the medicine into the interior of the air flow path when the user inhales said air stored in the container (2),
**characterized in that**
said container (2) has flexibility and comprises materials having low gas permeability, whereby said exhaled air of the user or said other air may maintain constant temperature and humidity; and
said medicine ejection part (4) uses the principle of an inkjet system.

2. The inhaler according to claim 1, which further comprises a detection means (5) for detecting the act of inhalation of the user.

3. The inhaler according to claim 2, wherein the detection means (5) contains a pressure sensor.

4. The inhaler according to any one of claims 1 to 3, wherein the gas holding part (2) is detachably attached to the flow path member (3).

## Patentansprüche

1. Inhalator zum Inhalieren von Medizin durch eine Saugöffnung, wobei der Inhalator Folgendes aufweist:
die Saugöffnung (6);
einen Behälter (2), um ausgeatmete Luft eines Anwenders oder andere Luft darin zu speichern;
ein Strömungspfadbauteil (3), das mit dem Behälter (2) verbunden ist und einen Luftströmungspfad zum Leiten der Medizin zu der Saugöffnung (6) durch Inhalieren des Anwenders ausbildet; und
ein Medizinausstoßteil (4) zum Ausstoßen der Medizin in das Innere des Luftströmungspfads dann, wenn der Anwender die in dem Behälter (2) gespeicherte Luft inhaliert,
**gekennzeichnet dadurch, dass**
der Behälter (2) Flexibilität aufweist und Materialien enthält, die eine niedrige Gasdurchlässigkeit haben, wodurch die ausgeatmete Luft des Anwenders oder die andere Luft konstante Temperatur und Feuchtigkeit beibehalten kann; und
bei dem Medizinausstoßteil (4) das Prinzip eines Tintenstrahlsystems zum Einsatz kommt.

2. Inhalator nach Anspruch 1, der des Weiteren eine Erfassungseinrichtung (5) zum Erfassen des Vorgangs des Inhalierens des Anwenders aufweist.

3. Inhalator nach Anspruch 2, wobei die Erfassungseinrichtung (5) einen Drucksensor enthält.

4. Inhalator nach einem der Ansprüche 1 bis 3, wobei der Gashalteteil (2) abnehmbar an dem Strömungspfadbauteil (3) angebracht ist.

## Revendications

1. Inhalateur destiné à l'inhalation d'un médicament via un orifice d'aspiration, l'inhalateur comprenant
l'orifice d'aspiration (6) ;
un récipient (2) destiné au stockage en son sein de l'air exhalé par un utilisateur ou d'un autre air ;
un élément formant passage d'écoulement (3) qui est relié au récipient (2) et forme un passage d'écoulement d'air pour guider le médicament jusqu'à l'orifice d'aspiration (6) par inhalation de l'utilisateur ; et
une partie d'éjection de médicament (4) pour éjecter le médicament à l'intérieur du passage d'écoulement d'air lorsque l'utilisateur inhale ledit air stocké dans le récipient (2),
**caractérisé en ce que**
ledit récipient (2) a une flexibilité et comprend des matériaux ayant une faible perméabilité aux gaz, grâce à quoi ledit air exhalé de l'utilisateur ou dudit autre air peut garder une température et une humidité constantes ; et
ladite partie d'éjection de médicament (4) utilise le principe d'un système à jet d'encre.

2. Inhalateur selon la revendication 1, qui comprend, en outre, un moyen de détection (5) pour détecter l'action d'inhalation de l'utilisateur.

3. Inhalateur selon la revendication 2, dans lequel le moyen de détection (5) renferme un capteur de pression.

4. Inhalateur selon l'une quelconque des revendications 1 à 3, dans lequel la partie contenant le gaz (2) est fixée, de manière amovible, à l'élément formant passage d'écoulement (3).
